# EUROPEAN PATENT APPLICATION

(11) **EP 2 540 279 A1**
(43) Date of publication of application: **02.01.2013**
(21) Application number: 11747017.9
(22) Date of filing: 22.02.2011
(51) Int. Cl.: A61K 8/92, A61K 8/37, A61Q 1/00

(54) **COATED POWDER AND COSMETIC PREPARATION USING THE COATED POWDER**

(30) Priority: 23.02.2010 JP 2010037563
(71) Applicant: Miyoshi Kasei, Inc., Saitama 3360975 (JP)
(72) Inventor: OHARA, Hiroyuki, Saitama-shi Saitama 336-0975 (JP); IMAZEKI, Masafumi, Saitama-shi Saitama 336-0975 (JP)
(74) Representative: Schmidt, Steffen
(86) International application number: PCT/JP2011/000968
(87) International publication number: WO 2011/105045

(57) **Abstract**

[PROBLEMS]

To provide a coated powder, which is free from a problem of generation of an unusual odor with the lapse of time when a powder is coated by using raw materials derived from plant fats and oils, can be blended into a cosmetic preparation, and has very smooth touch and excellent water repellency and pigment dispersibility. Cosmetic preparations containing such coated powders are also provided.

[Measures to solve the problems]

A coated powder is obtained by coating a powder to be treated, with a mixture, as a coating agent, which includes 60wt% to 90wt% of an extremely hardened plant fat and oil having the melting point of 50°C or more and 10wt% to 40wt% of an ester oil agent derived from a plant fat and oil. The powder is coated with the coating agent preferably at a temperature higher than the melting point of the extremely hardened plant fat and oil by 10 to 20°C. The powder is coated with the coating agent in an amount of 1wt% to 15wt%, particularly preferably 3wt% to 10wt%. Further, cosmetic preparations can be obtained by using the coated powder as a part or an entire part of the cosmetic preparations.

## Description

### [TECHNICAL FIELD]

The present invention relates to a coated powder characterized in that when a powder is coated by using a raw material derived vegetable fatty and oil, it does not suffer from a problem such as generation of an unusual odor with the lapse of time, has a very smooth touch and excellent water repellency and pigment dispersibility. The invention also relates to a cosmetic preparation into which this coated powder is blended.

### [Background Art]

In powders to be blended into conventional cosmetic preparations, many surface-treated powders having undergone the surface treatment are used. As surface-treating agents therefor, a fluorine compound, a silicone compound, an acylated amino acid, a fatty acid, an ester oil, an alkyl silane and the like are used. The surface treatments with these conventional powders have been made, mainly aiming at improving feeling on use (touch improvement), cosmetic effects, water repellency, and resultant long cosmetic-lasting performance (tenacity) modifications of the surfaces of the powders to afford functions such as easy blending in preparations (pigment dispersibility), etc.

However, as to raw materials to be recently blended into the cosmetic preparations, there are phenomena such as fear of fluorine compounds against the environment, departure from silicone compounds, departure from raw materials derived animals, etc. So, there is seen a tendency that they are shifted to raw materials derived plants. This tendency is similar in the case of the surface-treated powders, and it is expected that high-performance surface treatments are based on natural materials using plant natural raw materials.

Although there were conventionally surface treatments derived from the plant natural raw materials, surface treatments with plant extracts, cellulose or the like as surface-treating agents are predominant (for example, see Patent Document 1). In many cases, they are aimed at physiological effects and moisturizing effect, but they are insufficient for realizing the above-mentioned functions such as improvement on feeling on use, affording of water repellency, improvement on pigment dispersibility and the like.

On the other hand, surface treatments using hydrophobic plant materials have been being developed. For instance, there are surface treatments with plant fat and oil or plant waxes (For instance, See Patent Document 2). Although use of such materials as the surface-treating agents can afford hydrophobicity upon the surface-treated powders obtained, they are not functionally better as compared with conventional surface-treated powders.

Further, many raw materials derived from natural products including these plant fats and oils contain very small amounts of unsaturated fatty acids or the like. Even if powders are coated with the raw materials derived from plant fats and oils including such unsaturated fatty acids and the like, there is a very high possibility that the problems of the generation of the unusual odor with the lapse of time, etc. occur. It is feared that when such coated powders are blended into cosmetic preparations, qualities of the cosmetic preparations are remarkably deteriorated. Therefore, although it is desired that even the powders coated with the plant-origin raw materials such as plant fats and oils have excellent stability with the lapse of time, such powders have not been found in the market.

### [PRIOR ART DOCUMENTS]

### [PATENT DOCUMENTS]

Patent Document 1: JP-A 2009-046643
Patent Document 2: JP-A 2002-284642
Patent Document 3: JP-A 2007-129949
Patent Document 4: JP-A 2010-001366

### [SUMMARY OF THE INVENTION]

### [PROBLEMS TO BE SOLVED BY THE INVENTION]

Therefore, the object of the present invention is to provide a coated powder coated with raw materials derived from plant fats and oils, which coated powder is stable, does not cause unfavorable changes such as generation of unusual odor with the lapse of time and the like, has very smooth touch and are excellent in water repellency and dispersibility. The object of the present invention is also to provide a cosmetic preparation containing this coated powder.

### [MEASURES TO SOLVE THE PROBLEMS]

Having repeatedly made strenuous researches, the present inventors discovered that the above problems can be solved by coating a powder with a mixture of an extremely hardened plant fat and oil and an ester oil agent derived from a plant fat and oil, and the inventors have come to accomplish the present invention.

That is, the present invention relates to a coated powder characterized by being obtained by coating a powder to be coated, with a mixture of 60wt% to 90wt% of an extremely hardened plant fat and oil having a melting point of 50°C or more and 10wt% to 40wt% of a plant ester oil agent as a coating agent at a temperature higher than the melting point of the extremely hardened plant fat and oil by 10 to 20°C.

The following can be cited as preferred examples. Any combinations thereof are preferred examples of the present invention, if there is no particular contradiction.
(1) The coated powder is coated with the coating agent being the mixture such that the coating agent is in an amount of 1wt% to 15wt%, particularly 3wt% to 10wt%.
(2) The plant ester oil agent is a semisolid or a paste at ordinary temperatures.
(3) The extremely hardened plant fat and oil having the melting point of 50°C or more is at least one kind of extremely hardened plant fats and oils selected from the group consisting of extremely hardened camelia fat and oil, extremely hardened high-oleic sunflower oil, extremely hardened grape seed oil, extremely hardened rapeseed oil, extremely hardened high erucin rapeseed oil, extremely hardened macadamia nut oil, extremely hardened palm oil and extremely hardened soybean oil.
(4) The ester oil agent derived from the plant fat and oil is at least one kind of ester oil agents derived from plant fats and oils selected from the group consisting of hydroxystearic acid hydrogenated rucinus oil, isostearic acid hydrogenated rucinus oil, lauric acid hydrogenated rucinus oil, phytosteryl hydroxystearate, tri(caprylic acid/capric acid/myristic acid/stearic acid)glyceryl, bis(phytosteryl/behenyl/isostearyl)dimer dilinoleyl dimer dilinoleate, (phytosteryl/isostearyl/cetyl/stearyl/behenyl) dimer dilinoleate, phytosteryl macadamia nut fatty acid, and diethyl sebacate.
(5) The coated powder is a coated powder for cosmetic uses, which can be blended into a cosmetic preparation.

Furthermore, the present invention relates to a cosmetic preparation, wherein the coated powder in any of the above ones is blended at a part or an entire part of a powder component.

### [EFFECTS OF THE INVENTION]

The coated powder according to the present invention is stable, does not cause unfavorable changes such as generation of an unusual odor with the lapse of time and the like, has unprecedented and very smooth touch, and is excellent in water repellency and pigment dispersibility. The coated powder can be obtained, which can afford favorable characteristics on cosmetic preparations. The cosmetic preparation containing the coated powder according to the present invention has excellent stability and good stability with lapse of time, does not cause quality deterioration such as generation of unusual odor, etc., and exhibits very smooth touch, cosmetic effect lasting property and the like.

In the following, the contents of the present invention will be explained in detail.

### [EXAMPLES FOR CARRYING OUT THE INVENTION]

### (1) Indispensable ingredients

### (1-1) Extremely hardened plant fats and oils

It is indispensable to use the extremely hardened plant fat and oil as a first ingredient. Here, the extremely hardened plant fat and oil means the following extremely hardened plant fat and oil obtained by hydrogenating a plant fat and oil.

Patent Document 3 describes that there are broadly classified two kinds in types in the hydrogenated fats and oils. According to this publication, ones are extremely hardened fats and oils, and the other are partially hydrogenated fats and oils. The partially hydrogenated fat and oil contains a trans acid as an isomer (also referred to as trans type unsaturated fatty acid). Since that trans acid is an unsaturated fatty acid, there is a high possibility that a problem such as a change with lapse of time (generation of unusual odor), etc. occurs when heat treatment is carried out. In the United States of America, it is a duty since January 1, 2006 to indicate the contents of trans acids in foods, etc., because they increases a risk for myocardial infarction, and it is considered desirable that the trans acids are not contained in more than an amount present in nature. Further, it is said that the trans acids give anxiety in health. On the other hand, the extremely hardened plant fats and oils are those in which double bonds of the unsaturated fatty acid are hydrogenated until almost all unsaturated fatty acid contained in the plant fat and oil disappear, that is, until an iodine number reaches as near zero as possible. The extremely hardened plant fat and oil is a solid fat and oil which is excellent in heating stability and oxidation stability, and has a higher melting point as compared with the partially hydrogenated fats and oils.

The extremely hardened plant fats and oils to be used in the present invention need to have the melting points of 50°C or more. If the melting point is unfavorably 50°C or less, it can be melted during a producing process in the state that it is blended into a cosmetic preparation, so that coating may be peeled from the coated powder and water repellency may be lost. As the extremely hardened plant fats and oils having the melting point of 50°C or more, extremely hardened camelia fat and oil, extremely hardened high-oleic sunflower oil, extremely hardened grape seed oil, extremely hardened rapeseed oil, extremely hardened high erucin rapeseed oil, extremely hardened macadamia nut oil, extremely hardened palm oil, extremely hardened soybean oil and the like are recited, for example. The extremely hardened plant fats and oils are not limited to the above ones. The extremely hardened plant fat and oil can be produced by a generally known producing method. For example, according to Examples described in Patent Document 4, there is a method, for example, in which a plant fat and oil is hydrogenated by using a nickel catalyst, but the method is not limited thereto. As the extremely hardened fat and oil, a commercially available product can be used, too. As the commercially available product, there are a series of extremely hardened plant fats and oils manufactured by Yokozeki Oil & Fat Industries Co., Ltd and the like.

### (1-2) Ester oil agent derived from plant fats and oils

As a second ingredient, the present invention uses an ester oil agent derived from the plant fat and oil. "Derived from the plant fat and oil" means esters industrially produced from fatty acids obtained from fats and oils possessed by natural plants and alcohols. It is considered that the ester oil agent derived from the plant fat and oil functions to reduce a coefficient of friction and improve pigment dispersibility, etc. for the coated powder according to the present invention. As the ester oil agents derived from the plant fats and oils, no limitation is posed, so long as the object of the present invention is attained. Those which are liquid to solid at ordinary temperatures can be used, and those which are semisolid or paste at ordinary temperatures are more preferable. More preferably, those which have remarkable characteristics such as pigment dispersibility, emolliency, oxidation stability, etc. As ester oil agents derived from the plant fats and oils and preferred by the present invention, for example, mention may be made of hydroxystearic acid hydrogenated rucinus oil, isostearic acid hydrogenated rucinus oil, lauric acid hydrogenated rucinus oil, tri(caprylic acid/capric acid)glyceryl, polyglyceryl-2 tetraisostearic acid, polyglyceryl-2 isostearic acid, polyglyceryl-2 diisostearic acid, polyglyceryl-2 triisostearic acid, isostearic acid trehalose esters, phytosteryl hydroxystearate, phytosteryl oleic acid, tri(caprylic acid/capric acid/myristic acid/stearic acid)glyceryl, polyglyceryl-10 diisostearic acid, polyglyceryl-2 oleic acid, polyglyceryl-2 sesquicaprylic acid, sorbitan sesquioleate, sorbitan sesquiisostearate, glyceryl hydroxystearate, dimer dilinoleyl bis(phytosteryl/behenyl/isostearyl)dimer dilinoleate, (phytosteryl/isostearyl/cetyl/stearyl/behenyl) dimer dilinoleate, (isostearyl/phytosteryl) dimer dilinoleate, dimer dilinoleyl diisostealate, dimer dilinoleyl hydrogenated rosin condensate, diisostearyl malate, dimer dilinoleyl dimer dilinoleate, phytosteryl macadamia nut fatty acid, diethyl sebacate. cetyl ethylhexanoate isostearyl palmitate, trimethylolpropane triisostearate, triethylhexanoin, isopropyl myristate, diisopropyl sebacate, etc. However, the preferable ester oil agents are not limited thereto. As esters derived from plant fats and oils, Cosmol series manufactured by The Nisshin Oillio Group, Ltd. and the like are commercially available, and can be used, but the esters are not limited thereto. The ester oil agents derived from the plant fats and oils are preferably at least one kind of ester oil agents derived from the plant fats and oils selected from the group consisting of hydroxystearic acid hydrogenated rucinus oil, isostearic acid hydrogenated rucinus oil, lauric acid hydrogenated rucinus oil, phytostearyl hydroxystearate, tri(caprylic acid/capric acid/myristic acid/stearic acid)glyceryl, dimer dilinoleyl dimer dilinoleate bis(phytosteryl/behenyl/isostearyl), (phytosteryl/isostearyl/cetyl/stearyl/behenyl) dimer dilinoleate, phytosteryl macadamia nut fatty acid, and diethyl sebacate.

### (1-3) Mixing rate between the first ingredient and the second ingredient

The present invention is directed to the coated powder in which a powder to be usable in a cosmetic preparation is coated with the mixture, as the coating agent, of the above-mentioned extremely hardened plant fat and oil as the first ingredient and the ester oil agent derived the plant fat and oil as the second ingredient. It is important that the coating agent for the powder in the present invention is the mixture of 60wt% to 90wt% of an extremely hardened plant oil having the melting point of 50°C or more and 10wt% to 40wt% of a plant ester oil agent. "The mixture of 60wt% to 90wt% of the extremely hardened plant oil having the melting point of 50°C or more and 10wt% to 40wt% of the plant ester oil agent" means not only a case of the above two ingredients only but also a case where other ingredient is contained besides the first and second ingredients of the mixture, so long as the object, functions and effect of the present invention are not damaged.

When the powder is coated, while the mixing ratio of the extremely hardened plant fat and oil is set at 90wt% or more, touch is not smooth, and water repellency and pigment dispersibility become poorer. On the other hand, when the powder is coated in a state that the mixing rate is 60wt% or less, touch becomes sticky, and water repellency becomes poorer, so that desired functions can be hardly exhibited.

### (1-4) Powder-coating amount and coating temperature

The coating amount in which the powder as a base material is coated with the mixture of the extremely hardened plant fat and oil and the ester oil agent derived from the plant fat and oil as the coating agent differs depending upon the sizes and the specific surface area of the powder particles. However, if it is set at 1wt% to 15wt%, particularly preferably at 3wt% to 10wt%, the desired effects of the present invention can be exhibited to the utmost. When the powder is coated in an amount of less than 1wt% as a powder-coating amount, the effects of the present invention such as smooth touch and water repellency, pigment dispersibility and the like can hardly be fully attained. Furthermore, if the coating treatment is carried out in an amount of more than 15wt%, it is likely that the coated powder is flocculated, and further the functions of the powder may be damaged and it is also uneconomical.

In addition, it is preferable that the coating is carried out at a temperature higher than the melting point of the extremely hardened plant fat and oil by around 10°C to 20°C during the coating procedure. The ingredients may be decomposed at a higher temperature than the melting point of the mixture as the case may be, if the temperature is higher than the melting point of the mixture by more than 20°C, which deteriorates the object of the present invention. Meanwhile, if the coating treatment is carried out at a lower temperature, fluidity of the coating agent cannot be fully attained, so that a good coating condition does not tend to be obtained and consequently the effects of the present invention cannot be exhibited.

### (1-5) Powder to be coated

As the powder to be coated in the present invention, no particular limitation is posed, so long as it is a material to be used in the ordinary cosmetic preparations.

### (1-5a) Inorganic powder

For example, mention is made of as the inorganic powders, sericite, talc, mica, kaolin, synthetic mica, white mica, gold mica, synthetic gold mica, red mica, lithia mica, calcium carbonate, magnesium carbonate, calcium phosphate, alumina, magnesium oxide, aluminum hyroxide, barium sulfate, magnesium sulfate, silicic acid, silicic anhydride, magnesium silicate, aluminium silicate, aluminium magnesium silicate, calcium silicate, barium silicate, strontium silicate, silicon carbide, metal tungstate, magnesium aluminate, magnesium aluminometasilicate, chlorohydroxy aluminum, clay, bentonite, zeolite, smectite, hydroxyapatite, ceramic powder, boron nitride, boron nitride, silica and the like; as special body pigments in which a body pigment is combined, Excell mica, Excell pearl, Powder Lavie and the like put on sales by Miyoshi Kasei Kabushiki Kaisha; as white pigments, titanium oxide, zinc oxide, cerium oxide and the like; as coloring pigments, red iron oxide, yellow iron oxide, black iron oxide, chromium oxide, chromium hydroxide, iron blue, ultramarine blue, inorganic blue pigments, carbon black, low-functional titanium oxide, mango violet, cobalt violet, lake of tar colorant, lake of natural colorant and the like; as glittering pigments, bismuth oxychloride, titanium oxide-coated mica, argentine, a powder in which synthetic mica is coated with titanium oxide, powders put on sale as a trade name of "Meta Shine" from Nippon Sheet Glass Co. Ltd. in which silica flakes are coated with titanium oxide or the like, or in which alumina flakes are coated with tin oxide and titanium oxide, or in which aluminum flakes are coated with titanium oxide, powders put on sale by USA Eckert Co., Ltd. in which bronze flakes are coated with silica, or in which bronze flakes are coated with silica, or in which aluminum flakes are coated with silica, etc.; as particulate powder with the average particle sizes of less than 0.1µm, particulate titanium oxide, particulate zinc oxide, particulate iron oxide, particulate cesium oxide and the like; as powders having special shapes, butterfly-shaped barium sulfate, petal-shaped zinc oxide; as other powders, luminous powders put on sale as a trade name of "LumiNova series" from Mitsui & Co. Ltd., aluminum powder, stainless powder, tourmaline powder, amber powder and the like.

### (1-5b) Organic powders

Organic powders may be used, so long as they are not modified during a coating process with a coating agent in the present invention. For example, mention is made of synthetic resin powders such as wool powder, polyamide powder, polyester powder, polyethylene powder, polypropylene powder, polystyrene powder, polyurethane powder, benzoguanamine powder, polymethyl benzoguanamine powder, tetrafluoroethylene powder, poly(methyl metacrylate) powder, cellulose powder, silk powder, silicone powder, silicone rubber powder, styrene/acrylic acid copolymer, divinylbenzene/styrene copolymer, vinyl resin, urea resin, phenol resin, fluorine resin, silicon resin, acrylic resin, melamine resin, epoxy resin, polycarbonate resin and the like; powders such as finely crystalline fiber powder, starch powder, acylated lysine powder, long-chain alkyl metal phosphate powder, metallic soap powder, CI pigment yellow, CI pigment orange and the like. Further, as tar colors, use is made of powders of Red No. 3, Red No. 10, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 204, Green No. 205, Orange No 201, Orange No. 203, Orange No. 204, Orange No. 206, Orange No. 207 and the like; as natural colorants, carnine, laccaic acid, carthamin, brazilin, crocin and the like.

### (1-5c) Shapes, etc. of powders

Further, as shapes of powders to be used, various shapes such as plate-like shapes, spherical shapes, spindle-formed shapes, rod-like shapes, needle-like shapes, fibrous shapes, indeterminate shapes and the like can be used. In addition, no limitation is posed upon the sizes of the powder particles, so long as they are of sizes ordinarily used in cosmetics.

As these usable powders, mixtures in which two or more kinds of the above-mentioned powders are used and powders in the forms of composite bodies, attached shaped bodies can be used, so long as they can be mixed into ordinary cosmetics. Furthermore, complex or doped powders are involved in the usable powders, so long as they can be used in the cosmetics. As examples of them, powders in which an inorganic coloring pigment such as colcothar is coated with silicic anhydride, a powder in which nylon is coated with a white pigment, a powder in which a body pigment such as talc or the like is coated with a particulate white pigment, etc. are recited, but needless to say, the invention is not limited these examples.

In the present invention, powders already surface-treated with other chemical substances can be coated according to the present invention. For example, a powder treated with a metallic soap, a powder treated with silicone, a powder treated with fluorine, a powder treated with acylated amino acid, a coloring powder coated with a plant colorant, a powder coated with an ultraviolet absorber, powders coated with an antimicrobial ingredient or antioxidant and the like are coated according to the present invention. Needless to say, the invention is not limited to these examples.

### (2) Method for producing coated powders, etc.

In the present invention, when a powder is to be coated with a mixture of an extremely hardened plant fat and oil and an ester oil agent derived from a plant fat and oil as a coating agent, the coating treatment needs to be carried out at a temperature higher than the melting point of the extremely hardened plant fat and oil by around 10°C to 20°C. As the coating method, publicly known methods and machines generally used for modifying powders can be used, so long as surfaces of the powder can be ultimately coated with the coating agent.

As concretely treating methods for the coated powders according to the present invention, for example, mention may be made of methods in which a mixer such as a Henschel mixer equipped with a heatable heater or a jacket mechanism in which a heat medium can be circulated, a rocking mixer, a medial mill or the like is used, a method in which a slurry is obtained by adding a solvent such as isopropyl alcohol to a powder, a solution in which a coating agent in the present invention is solved is mixed thereinto and the solvent is distilled off under reduced pressured. When the mixer equipped with the jacket mechanism is to be used, a powder and a coating agent of the present invention (a mixture of an extremely hardened plant fat and oil and a ester oil agent derived from a plant fat and oil) are fed thereinto, heated and mixed, while steam is passed through the jacket so that the inside of the mixer may be at a temperature higher than the melting point of the extremely hardened plant fat and oil by 10 to 20°C. Thereby, the coated powder can be obtained. Note that the method for producing the coated powder according to the present invention is not limited to the method exemplified here.

Moreover, it is possible to combine powder-coating steps in the present invention during a process for producing a cosmetic preparation. More specifically, (1) a coated powder is first prepared, and a cosmetic preparation is produced in a separate process by using this coated powder. (2) A powder to be used in a cosmetic preparation is coated, which is used as a cosmetic as it is. Or, a cosmetic preparation is produced as a consecutive process by further adding a binder oil agent or the like thereto. Any of the producing processes can be selected.

### (3) Cosmetic preparations

The coated powder according to the present invention can be blended into a cosmetic preparation as a powdery ingredient. The cosmetic preparation according to the present invention can be obtained by combining one or two or more kinds of coated powders according to the present invention into a powder component. Alternatively, the coated powder according to the present invention can be blended as a part or an entire part of the powdery ingredient. At that time, the kind and the content of the coated powder to be blended into the cosmetic preparation are appropriately selected, depending upon the kind, the purpose, the form and the like of the cosmetic preparation. As to the kind, a use amount and a blending method, the preparation can be carried out by utilizing a method publicly known as a method for blending a powdery raw material into a cosmetic preparation or a method to be developed in the future.

As examples of cosmetic preparations into which the coated powders of the present invention can be blended, mention may be made of powdery cosmetic preparations such as white powder, powder eye shadow, cheek brusher, body powder, powder foundation and the like, aqueous liquid compositions such as liquid foundation, eye liner, mascara, carmine lotion, powder-containing lotion, milky liquid, cream and the like, various cosmetic preparations such as aqueous gel-like composition, emulsified composition, oily liquid composition, oily gel-like composition. The coated powder according to the present invention can be blended thereinto. No particular limitation is posed on cosmetic preparations into which the coated powders can be blended, so long as there is no problem on a producing process and a cosmetic composition. The invention is not limited to the above-examplified cosmetic preparations at all.

### [EXAMPLES]

The present invention will be explained more concretely by giving Examples and Comparative Examples below, but the technical scope of the present invention is not limited thereto.

### (Example 1 and Comparative Example 1)

An extremely hardened rapeseed oil (Yokozeki Oil & Fat Industries Co., Ltd.), 60.0wt% and lauric acid hydrogenated castor oil (Yokozeki Oil & Fat Industries Co., Ltd.), 40.0wt% were heated and mixed, and a solid mixture obtained by cooling was used as a coating agent. The melting point of the extremely hardened rapeseed oil used was 68°C. The coating agent, 3.09g was melted again, and fed into a table mixer equipped with a jacket together with 100g of sericite FSE (Sanshin Mining Ind. Co., Ltd.) . While the temperature inside the mixer was being kept at 85°C, mixing was carried out under stirring for 10 minutes, thereby obtaining 3wt% of a coated sericite powder as Example 1. Meanwhile, 60.0wt% of partially hydrogenated rapeseed fat and oil was used instead of the extremely hardened rapeseed fat and oil, and the same procedure as in the above was carried out by using a mixture, as a treating agent, in which the above partially hydrogenated rapeseed fat and oil was mixed with 40.0wt% of lauric acid hydrogenated castor oil under heating, thereby obtaining a coated powder as Comparative Example 1.

### (Example 2 and Comparative Example 2)

An extremely hardened rapeseed oil (Yokozeki Oil & Fat Industries Co., Ltd.), 70.0wt% and 30.0wt% of (phytosteryl/isostearyl/cetyl/stearyl/behenyl) dimer dilinoleate, (Nippon Fine Chemical Co., Ltd.) were mixed under heating, and a solid mixture obtained by cooling was used as a coating agent. The melting point of the used extremely hardened rapeseed oil was 68°C. The coating agent, 4.17g was melted again, and fed into a table mixer equipped with a jacket together with 100g of Talc JA-46R (ASADA MILLING CO.,LTD.), and the mixture was mixed under heating for 10 minutes, while the temperature inside the mixer was being kept at 85°C, thereby obtaining a 4wt%-coated talc powder as Example 2. Meanwhile, 70.0wt% of rapeseed oil (not hydrogenated) was used, which was mixed with 30.0wt% of (phytosteryl/isostearyl/cetyl/stearyl/behenyl) dimer dilinoleate under heating. The same procedure as in the above was carried out by using the mixture as a treating agent, thereby obtaining a coated powder as Comparative Example 2.

### (Example 3 and Comparative Example 3)

An extremely hardened macadamia nut oil (Yokozeki Oil & Fat Industries Co., Ltd.), 80.0wt% and 20.0wt% of tri(caprylic acid/capric acid/myristic acid/stearic acid)glyceryl (The Nisshin OilliO Group, Ltd., trade name: Salacos 334) were mixed under heating, and a solid mixture obtained by cooling was used as a coating agent. The melting point of the used extremely hardened macadamia nut oil was 59.4°C. The coating agent, 4.17g was melted again, and fed into a table mixer equipped with a jacket together with 100g of Mica M (Merk Japan Co., Ltd.), and the mixture was mixed under stirring for 10 minutes, while the temperature inside the mixer was being kept at 75°C, thereby obtaining a 4wt%-coated mica powder as Example 3. Meanwhile, 80.0wt% of the extremely hardened macadamia nut fat and oil and 20.0wt% of tri (caprylic acid/capric acid/myristic acid/stearic acid) glyceryl were mixed under heating. The same procedure as in the above was carried out by using the mixture as a treating agent, while the temperature inside the mixer was set at 55°C, thereby obtaining a coated powder as Comparative Example 3.

### (Example 4 and Comparative Example 4)

An extremely hardened macadamia nut fat and oil (Yokozeki Oil & Fat Industries Co., Ltd.), 90.0wt% and 10.0wt% of lauric acid hydrogenated castor oil (Yokozeki Oil & Fat Industries Co., Ltd.) were heated and mixed, and a solid mixture obtained by cooling was used as a coating agent. The melting point of the used extremely hardened macadamia nut fat and oil was 59.4°C. The coating agent, 51.26g, was melted again, and fed into a Henschel mixer equipped with a heating jacket together with 1kg of Mica Y-2000 (Yamaguchi Mica Co., Ltd.), and the mixture was mixed under stirring for 10 minutes, while the temperature inside the mixer was being kept at 75°C, thereby obtaining a 5wt%-coated mica powder as Example 4. Meanwhile, 30.0wt% of the extremely hardened macadamia nut fat and oil and 70.0wt% of lauric acid hydrogenated castor oil (Yokozeki Oil & Fat Industries Co., Ltd.) were heated and mixed, and the same procedure as in the above was carried out by using the mixture as a treating agent, thereby obtaining a coated powder as Comparative Example 4.

### (Example 5 and Comparative Example 5)

An extremely hardened macadamia nut fat and oil (Yokozeki Oil & Fat Industries Co., Ltd.), 80.0wt% and 20.0wt% of tri(caprylic acid/capric acid/myristic acid/stearic acid)glyceryl (The Nisshin OilliO Group, Ltd., trade name: Salacos 334) were heated and mixed, and a solid mixture obtained by cooling was used as a coating agent. The melting point of the used extremely hardened macadamia nut fat and oil was 59.4°C. The coating agent, 52.63g, was melted again, and fed into the Henschel mixer equipped with the heating jacket together with 1kg of Mica Y-2000 (Yamaguchi Mica Co., Ltd.), and the mixture was mixed under stirring for 10 minutes, while the temperature inside the mixer was being kept at 75°C, thereby obtaining a 5wt%-coated mica powder as Example 5. Meanwhile, 80.0wt% of the extremely hardened macadamia nut fat and oil and 20.0wt% of vaseline were mixed under heating, and the same procedure as in the above was carried out by using the mixture as a treating agent, thereby obtaining a coated powder as Comparative Example 5.

### (Example 6 and Comparative Example 6)

An extremely hardened palm oil (Yokozeki Oil & Fat Industries Co., Ltd.), 75.0wt% and 25.0wt% of phytosteryl macadamia nut fatty acid (Nippon Fine Chemical Co., Ltd., trade name: YOFCO MAS)) were heated and mixed, and a solid mixture obtained by cooling was used as a coating agent. The melting point of the extremely hardened palm oil was 59°C. A slurry was obtained by adding isopropyl alcohol to 100g of particulate titanium oxide MT-100SA (manufactured by Tayca Corporation), and the temperature was set at 80°C. To the slurry was added 6.23g of the re-melted coating agent. After mixing under stirring, a coated powder was obtained by distilling off the isopropyl alcohol under reduced pressure, and pulverized by a jet mill (manufactured by Hosokawa Micron Corporation), thereby obtaining a 6wt%-coated particulate titanium oxide powder as Example 6. Meanwhile, 75.0wt% of an extremely hardened palm oil and 25.0wt% of vaseline were heated and mixed, and the same procedure as in the above was carried out by using the mixture as a treating agent, thereby obtaining a coated powder as Comparative Example 6.

### (Example 7 and Comparative Example 7)

An extremely hardened palm oil (Yokozeki Oil & Fat Industries Co., Ltd.), 70.0wt% and 30.0wt% of tri(caprylic acid/capric acid/myristic acid/stearic acid)glyceryl (The Nisshin OilliO Group, Ltd., trade name: Salacos 334) were heated and mixed, and a solid mixture obtained by cooling was used as a coating agent. The melting point of the used extremely hardened palm fat and oil was 59°C. The coating agent, 4.17g, was melted again, and mixed with 100g of titanium oxide CR-50 (Ishihara Sangyou Kabushiki Kaisha) were mixed under stirring for 10 minutes by the table mixer with the jacket, while the temperature inside the mixer was kept at 75°C, thereby obtaining a 4wt%-coated titanium oxide powder as Example 7. Meanwhile, titanium oxide CR-50 was mixed with methyl hydrogen polysiloxane as a generally used hydrophobically surface-treating agent such that a treating amount might be 2.0wt%, and they were reacted under heating at 115°C for 3 hours, thereby obtaining a silicone-treated titanium oxide powder as Comparative Example 7.

### (Evaluation method)

Next, methods which were used for evaluations of Examples 1 to 7 and Comparative Examples 1 to 7 will be explained.

### (1) Evaluation of generation of odors with lapse of time

Each of the coated powders obtained in Examples 1 to 7 and Comparative Examples 1 to 7 was put in a transparent bottle with a threaded mouth, which was left at 50°C. Two weeks later from the coating treatment, odors were subjected to sensory evaluations through comparison by five persons in a special panel. At a time when an unusual odor was felt, judgment was performed according to an evaluation standard shown below.
O: No unusual odor was felt even after 2 weeks.
Δ : Although no unusual odor was felt after the treatment, a usual odor was fed 2 weeks later.
X: An unusual odor was felt from after the treatment.

### (2) Evaluation of hydrophobicity

Into a 100ml beaker was put 80ml of water, and 0.5g of each of the coated powders was placed on a surface of the water. After it was left stationary for 1 hour, it was evaluated whether the coated powder moved into the aqueous phase or not. An evaluation standard is as follows.
5: The coated powder is kept floated on the water surface, and no powder moves into the aqueous phase.
4: Although the powder is kept floated, the water is slightly clouded.
3: Although the powder is kept floated, the water is clouded.
2: Although the powder is kept floated, the water is adequately clouded.
1: All the powder moves into the aqueous phase, and a precipitation occurs.

### (3) Sensory evaluations: Coating touch and coated state

A coated powder in each of Examples and Comparative Examples was taken with a puff by 5 persons in a special panel, touch and coated state when coated on an inner side portion of an upper arm were relatively evaluated through being compared. Each of Examples and Comparative Examples was scored according to a scale of 1 to 5: good touch (5 points) to bad touch (one point), or according to a scale of 1 to 5: uniformly coated state (5 points) to coarsely coated state (one point), and sensory evaluations of the coated touch and the coated state were performed by the average value in 5 persons (figures after the decimal point being rounded down)

A list evaluation results in Examples 1 to 7 and Comparative Examples 1 to 7 was given in Table 1.
[Table 1]

**Table 1**

| | Generation of unusual odor over time | Hydrophobicity | Touch of coating | Coated state |
|---|---|---|---|---|
| Example 1 | O | 4 | 5 | 5 |
| Example 2 | O | 5 | 4 | 5 |
| Example 3 | O | 5 | 5 | 5 |
| Example 4 | O | 5 | 4 | 4 |
| Example 5 | O | 5 | 5 | 4 |
| Example 6 | O | 5 | 4 | 5 |
| Example 7 | O | 5 | 4 | 4 |
| Com. Ex. 1 | Δ | 3 | 4 | 3 |
| Com. Ex. 2 | X | 1 | 2 | 1 |
| Com. Ex. 3 | O | 2 | 1 | 1 |
| Com. Ex. 4 | O | 3 | 2 | 2 |
| Com. Ex. 5 | O | 4 | 2 | 1 |
| Com. Ex. 6 | O | 3 | 3 | 1 |
| Com. Ex. 7 | O | 5 | 3 | 3 |

As clear from Table 1, the coated powders in Examples 1 to 7 showed better results than Comparative Examples. As to the coated powders in Comparative Examples 1 and 2 using the partially hydrogenerated fat and oil and the plant fat and oil (not hydrogenated), an unusual odor was felt with lapse of time, and hydrophobicity was weaker. On the other hand, in any of Examples, the stability was high, and the hydrophobicity was high.

In addition, with respect to the coated powders in Examples 1 to 7 and Comparative Examples 1 to 7, a coefficient of dynamic friction and a contact angle were measured according to the following methods, and more detailed evaluations of touch and water repellency at the time of coating were performed.

### (4) Coefficient of dynamic friction

Each coated powder is coated onto a 8cm x 5cm piece of a synthetic leather (Idemitsu Kosan Co., Ltd., trade name: Supplale) under condition of 1mg/cm2, which is set in a dynamic friction coefficient measuring tester (Shinto Scientific Co., Ltd., TRIBOSTATION Type 32). A non-coated artificial leather is placed thereon such that their faces may be opposed to each other. Then, a load of 50g/cm2 was applied thereon and the leathers were reciprocated three times. The average value of the coefficients of dynamic friction was determined. The smaller the figure, the smoother is the coated surface.

### (5) Measurement of contact angle

Each of the coated powders was coated onto a synthetic lather similarly as in the evaluation of the coefficient of dynamic friction, and a 2µL drop of distilled water was fallen thereon. An angle formed between a surface of the synthetic leather and a tangent of the formed water drop was determined as a contact angle. The magnitude of the contact angle was taken as the intensity of water repellency.

Results of the coefficients of dynamic frictions and the contact angles in Examples 1 to 7 and Comparative Examples 1 to 7 were given in Table 2. [Table 2]

**Table 2**

| | Coefficient of dynamic friction | Contact angle (°) |
|---|---|---|
| Example 1 | 0. 354 | 141. 7 |
| Example 2 | 0. 334 | 130. 0 |
| Example 3 | 0. 326 | 124. 7 |
| Example 4 | 0. 311 | 123. 7 |
| Example 5 | 0. 326 | 127. 7 |
| Example 6 | 0. 329 | 129. 0 |
| Example 7 | 0. 365 | 137. 0 |
| Com. Ex. 1 | 0. 678 | 49. 3 |
| Com. Ex. 2 | 0. 702 | 48. 8 |
| Com. Ex. 3 | 0. 699 | 85. 3 |
| Com. Ex. 4 | 0. 665 | 118. 3 |
| Com. Ex. 5 | 0. 754 | 100. 7 |
| Com. Ex. 6 | 0. 775 | 103. 7 |
| Com. Ex. 7 | 0. 516 | 134. 7 |

As clear from Table 2, it is shown that the coated powders in Examples 1 to 7 have smaller coefficients of dynamic friction, high smoothness and excellent touch. In addition, they had large contact angles. From the results in Example 7 and Comparative Example 7, the coated powder showed equivalent water repellency as compared with a silicone-treated powder used as a water-repellent treated powder in general cosmetic preparations.

### (Example 8 and Comparative Example 8)

A base material.of yellow iron oxide (Titan Kogyo, Ltd.) was coated according to the same coating method as in Example 1, thereby obtaining a coated powder in Example 8. Meanwhile, a powdery base material of the same yellow iron oxide was surface-treated in the same treating amount as in Examples of a surface-treating method described in JP-A 58-72512, thereby obtaining a stearoyl glutaminate-treated powder In Comparative Example 8.

### (Example 9 and Comparative Example 9)

A coated powder in Example 9 was obtained by coating colcothar (Titan Kogyo, Ltd.) as a base material according to the same coating method as in Example 1. Meanwhile, a stearic acid-coated powder in Comparative Example 9 was obtained by surface-treating colcothar as a base material in the same treating amount as in Examples according to a surface-treating method described in JP-A 60-69011.

### (Example 10 and Comparative Example 10)

A coated powder in Example 10 was obtained by coating black iron oxide (Titan Kogyo, Ltd.) as a base material according to the same coating method as in Example 1. Furthermore, an ester-treated powder in Comparative Example 10 was obtained by surface-treating the same black iron in the same treating amount as in Examples according to a surface-treating method described in JP-A 2004-51945.

With respect to Examples 7 to 10 and Comparative Examples 7 to 10, dispersibility was evaluated according to the following method.

### (6) Dispersibility

Each of the coated powders, 1g, was added to 3g of an acryl silicone oil (Shin-Etsu Chemical Co., Ltd.: KP-545), the mixture was well mixed, around 1g thereof was dropped on a contrast ratio-measuring paper (manufactured by Motofuji Co., Ltd.), and a coated film was formed by a bar coater (manufactured by Tester Sangyo Co., Ltd., ROD No. 12, film thickness 27.4µm), and dried at room temperature over a day and a night. The coated film on a whitish face of the contrast ratio-measuring paper was visually evaluated, and dispersibility was evaluated according to the following standard.

### (Dispersibility-evaluating standard)

5: Coated film is uniform, and presence of particles is not felt.
3: Although coated film is uniform, presence of slight particles is felt.
1: Coated film is non-uniform, and presence of particles is strongly felt.

With respect to Examples 7 to 10 and Comparative Examples 7 to 10, evaluation results of the dispersibility were shown in Table 3. [Table 3]

**Table 3**

| | dispersibility |
|---|---|
| Example 7 °C | 5 |
| Com. Ex. 7 | 5 |
| Example 8 | 5 |
| Com. Ex. 8 | 1 |
| Example 9 | 5 |
| Com. Ex. 9 | 3 |
| Example 1 0 | 5 |
| Com. Ex. 1 0 | 3 |

As is clear from Table 3, it is seen that the coated powders according to the present invention have high performance as compared with the surface-treated powders as having been used in conventional cosmetic preparations.

Next, cosmetic preparations containing the coated powders in Examples are prepared, and effects of the coated powders according to the present invention are further verified.

### (Example 11) (Powder foundation)

A powder foundation was produced as follows.
[Table 4]

**Table 4**

| Ingredient | | Mixing rate (wt%) |
|---|---|---|
| 1 | Coated sericite powder in Example 1 | 36. 84 |
| 2 | Coated talc powder in Example 2 | 20. 00 |
| 3 | Coated mica powder in Example 5 | 35. 00 |
| 4 | Coated titanium oxide powder in Example 7 | 3. 00 |
| 5 | Coated yellow iron oxide powder in Example 8 | 0. 10 |
| 6 | Coated colcothar powder in Example 6 | 0. 06 |
| 7 | Hydrogenated polyisobutene | 3. 00 |
| 8 | Dimethyl polysiloxane (20cst) | 1. 50 |
| 9 | Vaseline | 0. 50 |

### (Producing method)

Powder raw materials: ingredients 1 to 6 are mixed in a Henschel mixer and pulverized by an atomizer. Ingredients 7 to 9 are mixed and melted under heating, which is added into the Henschel mixer where the mixture is further mixed. The mixture is pulverized again by the atomizer. The pulverized mixture was compression molded in a medium-sized plate, thereby obtaining a powder foundation.

### (Comparative Example 11]

A powder foundation in Comparative Example 11 was produced by the same procedure as in Example 11 except that the powder ingredients 1 to 6 in Example 11 were replaced by a silicone-coated powder obtained by the same method as in Comparative Example 7.

### (7) Sensory evaluations: evaluations on touch, color saturation, cosmetic finish and cosmetic long-lasting performance

With respect to the powder foundations produced in Example 11 and Comparative Example 11, touch, color saturation, cosmetic finish and cosmetic long-lasting performance were evaluated by sensor evaluations with 5 persons in a special panel. In addition, the above-mentioned stability with the lapse of time (odor) was repeatedly evaluated. Results are summarized in Table 5, and an evaluation standard is as follows.
(Sensory evaluation standard)
5: Very good
4: somewhat good
3: Medium
2: Rather worse 1
1: Largely bad

[Table 5]

**Table 5**

| Evaluation item | Example 1 1 | Com. Ex. 1 1 |
|---|---|---|
| Touch | 5 | 4 |
| Color saturation | 5 | 4 |
| Cosmetic finish | 5 | 3 |
| Cosmetic long lasting performance | 5 | 4 |
| Stability over time (rancid) | 5 | 5 |

From the evaluation results in Table 5, the powder foundation containing the coated powder according to the present invention is excellent in touch, color saturation and cosmetic finish, and also excellent in cosmetic long-lasting performance and stability with lapse of time (odor).

### (Example 12])(Liquid foundation)

A liquid foundation was produced as follows.
[Table 6]

**Table 6**

| Ingredient | | Mixed ratio (wt%) |
|---|---|---|
| 1 | Coated titanium oxide powder in Example 7 | 8. 00 |
| 2 | Coated yellow iron oxide powder in Example 8 | 2. 04 |
| 3 | Coated colcothar powder in Example 9 | 0.34 |
| 4 | Coated black iron oxide powder in Example 10 | 0. 24 |
| 5 | Cyclopentasiloxane | 22. 93 |
| 6 | Dimethyl polysiloxane (20cst) | 10.00 |
| 7 | Ethylhexyl methoxycinnamate | 2. 00 |
| 8 | (Dimethicone/vinyldimethicone)crosspolymer | 2. 00 |
| 9 | Distear dimonium hectorite | 1 . 00 |
| 10 | PEG-10 Dimetycon P E G - 1 0 dimethicone | 3. 00 |
| 11 | Purified water | 43. 00 |
| 12 | BG | 5. 00 |
| 13 | Methylparaben | 0. 15 |
| 14 | Phenoxyethanol | 0. 30 |

### (Producing method)

Coated powders in Ingredients 1 to 4 are uniformly mixed, and pulverized in a pulverizer. Next, Ingredients 5 to 10 are mixed, swelled, and sufficiently homogenized by using a homogenizer. Then, the powder component is added thereto, which is uniformly dispersed in the homogenizer to obtain an oil phase. An aqueous phase is obtained by adding Ingredients 11 to 14 and uniformly dissolving them. While the oil phase is being stirred in the homogenizer, the aqueous phase component is gradually added to form an emulsion, thereby obtaining a liquid foundation.

The liquid foundation produced in Example 12 was put in a transparent bottle with a threaded mouth. It was kept stationary at 50°C similarly in the above evaluation for stability with lapse of time, and the stability over time was evaluated 2 weeks later. The liquid foundation in Example 12 generated no unusual odor with the lapse of time, and showed a good state over time without color separation, flocculation of the powder ingredients or the like. In addition, Sensory evaluations were carried out similarly as in Example 11 by a special panel, which consequently showed that the liquid foundation had a good finish.

As having been explained above in detail, the coated powder according to the present invention, which is coated by using a mixture of the extremely hardened plant fat and oil having the melting point of 50°C or more and the ester oil agent derived from the plant fat and oil mixed at a mixing ratio of 60wt% to 90wt% of the extremely hardened plant fat and oil and 10wt% to 40wt% by weight of the ester oil agent under the condition that the coating temperature is higher than the melting point of the extremely hardened plant fat and oil by around 10°C to 20°C is excellent in touch, water repellency, pigment dispersibility and luster with generation of no unusual odor, as compared with the coated powders coated by using the partially hydrogenated plant fat and oil or the plant oil. Further, the coated powder according to the present invention has more excellent performances in touch and color saturation (pigment dispersibility) as compared with surface-treated powders, such as silicone-treated ones, having been used in conventional cosmetic preparations. Thus, the coated powder according to the present invention is ideal as a powder for cosmetic preparations, which is to be blended into makeup cosmetic preparations represented by powder foundation, liquid foundation, pressed powder, eye shadow, blusher, face powder and the like.

## Claims

1. A coated powder **characterized by** being obtained by coating a powder to be coated, with a mixture of 60wt% to 90wt% of an extremely hardened plant fat and oil having a melting point of 50°C or more and 10wt% to 40wt% of a plant ester oil agent as a coating agent

2. The coated powder set forth in claim 1, wherein the powder to be treated is coated with the coating agent at a temperature higher than the melting point of the extremely hardened plant fat and oil by 10 to 20°C.

3. The coated powder set forth in claim 1 or 2, wherein the powder is coated with the coating agent being the mixture such that the coating agent is in an amount of 1wt% to 15wt% to the powder.

4. The coated powder set forth in claim 3, wherein the powder is coated with the coating agent being the mixture such that the coating agent is in an amount of 3wt% to 10wt% to the powder.

5. The coated powder set forth in any one of claims 1 to 4, wherein the plant ester oil agent is a semisolid or a paste at ordinary temperatures.

6. The coated powder set forth in any one of claims 1 to 5, wherein the extremely hardened plant fat and oil having the melting point of 50°C or more is at least one kind of extremely hardened plant fats and oils selected from the group consisting of extremely hardened camelia fat and oil, extremely hardened high-oleic sunflower oil, extremely hardened grape seed oil, extremely hardened rapeseed oil, extremely hardened high erucin rapeseed oil, extremely hardened macadamia nut oil, extremely hardened palm oil and extremely hardened soybean oil.

7. The coated powder set forth in any one of claims 1 to 6, wherein the ester oil agent derived from the plant fat and oil is at least one kind of ester oil agents derived from plant fats and oils selected from the group consisting of hydroxystearic acid hydrogenated rucinus oil, isostearic acid hydrogenated rucinus oil, lauric acid hydrogenated rucinus oil, phytosteryl hydroxystearate, tri(caprylic acid/capric acid/myristic acid/stearic acid)glyceryl, bis(phytosteryl/behenyl/isostearyl)dimer dilinoleyl dimer dilinoleate, (phytosteryl/isostearyl/cetyl/stearyl/behenyl)dimer dilinoleate, phytosteryl macadamia nut fatty acid, and diethyl sebacate.

8. The coated powder set forth in any one of claims 1 to 7, wherein the coated powder is a coated powder for cosmetic uses, which can be blended into a cosmetic preparation.

9. A cosmetic preparation, wherein the coated powder in any one of claims 1 to 8 is blended as a part or an entire part of a powder component.
